# EUROPÄISCHE PATENTANMELDUNG

(11) **EP 3 566 923 A1**
(43) Veröffentlichungstag der Anmeldung: **13.11.2019**
(21) Anmeldenummer: 19168092.5
(22) Anmeldetag: 09.04.2019
(51) Int. Cl.: B60W 50/00, B60W 50/08, B60W 50/10, B60W 40/08

(54) **VERFAHREN UND STEUEREINHEIT ZUM BETREIBEN EINES AUTONOMEN FAHRZEUGS**

(30) Priorität: 07.05.2018 DE 102018207069
(71) Anmelder: ZF Friedrichshafen AG, 88046 Friedrichshafen (DE)
(72) Erfinder: Stauber, Tobias, 88048 Friedrichshafen (DE)

(57) **Zusammenfassung**

Die Erfindung betrifft eine Steuereinheit für autonomes Fahren (18), die einen Prozessor(40), der dazu ausgelegt ist, ein durchgeführtes oder ein anstehendes Fahrmanöver (M1-M4) auf Grundlage von physiologischen Messwerten zu bewerten.

## Beschreibung

### TECHNISCHES GEBIET

Die vorliegende Offenbarung betrifft ein Verfahren und eine Steuereinheit zum Betreiben eines autonomen Fahrzeugs.

### TECHNISCHER HINTERGRUND

Als autonomes Fahrzeug bezeichnet man ein Fahrzeug, das ohne Einfluss eines menschlichen Fahrers fahren, steuern und einparken kann. Beim autonomen Fahren übernimmt das Steuerungssystem des Fahrzeugs vollständig oder weitestgehend die Rolle des Fahrers. Autonome Fahrzeuge können mit Hilfe verschiedener Sensoren ihre Umgebung wahrnehmen, aus den gewonnenen Informationen ihre Position und die anderer Verkehrsteilnehmer bestimmen und mithilfe des Steuerungssystems und der Navigationssoftware des Fahrzeugs das Fahrziel ansteuern und im Straßenverkehr entsprechend agieren.

Wie aus der Schrift DE 10 2014 212 746 A1 hervorgeht, hat die Verwendung von Automation beim Fahren von Straßenfahrzeugen, wie Autos und Lastwagen, durch die Fortschritte in Sensortechnologien (z.B. Objektnachweis und Standortverfolgung), Kontrollalgorithmen und Dateninfrastrukturen zugenommen.

Neben dem Gewinn an Mobilität, vor allem für Menschen mit Behinderung und ältere Menschen, reduziert automatisiertes Fahren die Gefahr von Unfällen durch zu lange Reaktionszeiten, Müdigkeit, Ablenkung und andere menschliche Faktoren.

Andererseits können autonome (selbstfahrende) Fahrzeuge ein Fahrverhalten aufweisen, das sich vom Fahrverhalten von Personen-gesteuerten Fahrzeugen deutlich unterscheidet, zum Beispiel was das Bremsverhalten und das Manövrieren im Straßenverkehr betrifft. So können manche Benutzer und Fahrgäste autonomer Fahrzeuge die Reaktionen des Fahrzeugs als unerwartet, unangenehm oder sogar beängstigend empfinden.

Vor diesem Hintergrund offenbart die internationale Patentanmeldung WO 2016/070981 A1 ein System und ein Verfahren zur Überwachung des Gesundheitszustandes eines Fahrzeuginsassen. Das System umfasst eine Steuereinheit umfassend einen Empfänger zum drahtlosen Empfangen von physiologischen Parametern von mindestens einer am Körper zu tragenden Einheit, welche einen oder mehrere Sensoren zum Ermitteln von einem oder mehreren physiologischen Parametern des Fahrzeuginsassen umfasst, und ein Diagnosemodul, das dazu eingerichtet ist, zumindest teilweise basierend auf den empfangenen physiologischen Parametern Information bezüglich des Gesundheitszustandes, des Befindenszustandes oder von krankhaften Ereignissen abzuleiten. Die Steuereinheit ist weiterhin dazu eingerichtet, den Fahrzeuginsassen über mindestens eine Ausgabeeinheit des Fahrzeugs über den Gesundheitszustand zu informieren und mindestens einen der folgenden Schritte einzuleiten: Fahrzeugfunktionen an den Zustand anzupassen, oder über mindestens eine Ausgabeeinheit des Fahrzeugs Maßnahmen vorzuschlagen oder interaktiv durchzuführen, die der Verbesserung des Zustandes dienen.

Hiervon ausgehend liegt der Erfindung die Aufgabe zu Grunde, ein Verfahren und eine Steuerungseinheit zum Betreiben eines autonomen Fahrzeugs bereitzustellen, das das Fahrverhalten des Fahrzeugs optimiert.

Diese Aufgabe wird durch die Steuereinheit für autonomes Fahren nach Anspruch 1 und das Verfahren nach Anspruch 10 gelöst. Weitere vorteilhafte Ausgestaltungen der Erfindung ergeben sich aus den Unteransprüchen und der folgenden Beschreibung bevorzugter Ausführungsbeispiele der vorliegenden Erfindung.

Gemäß den unten beschriebenen Ausführungsbeispielen wird eine Steuereinheit für autonomes Fahren bereitgestellt, die einen Prozessor umfasst, der dazu ausgelegt ist, ein durchgeführtes oder ein anstehendes Fahrmanöver auf Grundlage von physiologischen Messwerten zu bewerten.

Bei der Steuereinheit für autonomes Fahren kann es sich beispielsweise um ein Steuergerät (engl. ECU = electronic control unit oder ECM = electronic control module) handeln. Die Steuereinheit für autonomes Fahren (z.B. ein "Autopilot") kann beispielsweise in einem autonomen Fahrzeug eingesetzt werden, so dass dieses ganz oder teilweise ohne Einfluss eines menschlichen Fahrers fahren, steuern und einparken kann. Die Steuereinheit kann sich im Fahrzeug befinden, oder auch außerhalb oder teilweise außerhalb des Fahrzeugs. So kann es sich beispielsweise um einen Algorithmus handeln, der auf einem Server oder einem Cloud-System abläuft. Beispielsweise können Messwerte in einem Fahrzeug ermittelt werden und von dort zu einem Server oder in ein Cloud-System übertragen werden, wo auf Grundlage der übermittelten Messwerte das Befinden der Fahrzeuginsassen ermittelt und das Ergebnis wieder an das Fahrzeug rückübermittelt wird. Zusätzlich zu den Messwerten könnte das Fahrzeug auch mögliche Fahrmanöver an den Server bzw. das Cloud-System übermitteln, und eine Bewertung der Fahrmanöver auf Grundlage der Messwerte bzw. der Befindlichkeit der Fahrzeuginsassen könnte auf dem Server bzw. in der Cloud stattfinden. Auch könnte die Auswahl des optimalen Fahrmanövers außerhalb des Fahrzeugs, auf dem Server oder in der Cloud stattfinden, so dass dem autonomen Fahrzeug lediglich das Aufgrund der Messwerte ausgewählte Fahrmanöver übermittelt wird. Demgemäß ist vorgesehen, dass die Steuereinheit bzw. Steuerlogik ganz oder teilweise auch außerhalb eines Fahrzeugs liegen kann.

Bei dem Prozessor kann es sich beispielsweise um eine Recheneinheit wie eine zentrale Verarbeitungseinheit (CPU = central processing unit) handeln, die Programminstruktionen ausführt.

Bei einem Fahrmanöver kann es sich beispielsweise um eine bestimmte Fahrsituation handeln, die eine objektiv gegebene räumliche und zeitliche Konstellation der verkehrsbezogenen Einflussgrößen der Funktionsumgebung eines Fahrzeugs darstellt. Fahrmanöver können in der Steuereinheit vordefiniert sein und beispielsweise durch Kontextinformationen (z.B. Position des Fahrzeugs, Navigationskontext oder dergleichen) und Fahrzeugbetriebsparameter (Geschwindigkeit, Querbeschleunigung, Drehmoment) festgelegt sein. Fahrmanöver können nach bestimmten Kategorien klassifiziert sein. Beispiele für Fahrmanöver sind "Abfahren von einer Autobahn", "Fahrt durch eine Kurve", "Annähern an eine Ampel", "Auffahren auf den Vordermann", "Abstand zum Fahrbahnrand", "Anbremsen einer Kurve", "Überholen auf der Autobahn, "Überholen auf einspurigen Straßen", "Beschleunigen auf Auffahrten", "Verzögern auf Abfahrten", "Anfahren", Fahren über Bodenwellen oder Bremsschwellen, und dergleichen. Fahrmanöver einer Kategorie können in verschiede Fahrmanövervarianten unterteilt sein, wie beispielsweise "Fahrt durch eine Kurve mit einer Querbeschleunigung von 2 m/s²" als eine erste Variante, "Fahrt durch eine Kurve mit einer Querbeschleunigung von 3 m/s²" als eine zweite Variante, usw. Statt nach Querbeschleunigung könnte das Fahrmanöver auch bezüglich der Größen Kurvenradius und Fahrgeschwindigkeit in Fahrmanövervarianten aufgeteilt werden. Solche Fahrmanöver werden von jedem Menschen anders wahrgenommen und als angenehm oder unangenehm bewertet.

Die Steuereinheit kann dazu ausgelegt sein, das Fahrzeug zumindest teilweise auf Grundlage einer personenspezifischen Bewertung eines Fahrmanövers zu steuern und somit das Fahrverhalten des Fahrzeugs zu optimieren. Beispielsweise kann der Prozessor der Steuereinheit für autonomes Fahren dazu ausgelegt sein, ein durchgeführtes oder ein anstehendes Fahrmanöver zu bewerten, indem einem Fahrmanöver auf Grundlage von physiologischen Messwerten bezüglich eines Fahrzeuginsassen eine Bewertung zugeordnet wird. Die Bewertung kann beispielsweise durch einen Parameter, einen Zahlenwert, oder dergleichen definiert sein. Beispielsweise können verschiedene Bewertungen in der Steuereinheit für autonomes Fahren vordefiniert sein, wobei jede Bewertung einer Befindlichkeit eines Fahrzeuginsassen zugeordnet ist.

Die physiologischen Messwerte können sich beispielsweise auf einen Insassen eines autonomen Fahrzeugs beziehen, zum Beispiel einen Fahrzeugführer oder aber auch auf weitere Insassen, wie einen Beifahrer oder Personen auf der Rückbank des Fahrzeugs. Beispielsweise kann ein Fahrmanöver darin bestehen, dass ein autonomes Fahrzeug an einer roten Ampel bremst. Dieses Fahrmanöver kann ausgeführt werden, indem das Fahrzeug bereits weit vor der roten Ampel abbremst, "rollt" (in Schub geht), rekuperiert oder "segelt" (Fahrzeugantriebsstrang offen Kupplung offen) und das Fahrzeug damit verzögert wird. Springt die Ampel auf Grün um, muss das Fahrzeug wieder beschleunigt werden, damit es noch während der Grünphase die Kreuzung passieren kann. Dies könnte dazu führen, dass die Insassen des Fahrzeugs über das frühzeitige Verzögern und anschließende Beschleunigen nicht erfreut sind und mit Stress reagieren. Die vorliegende Steuereinheit kann solche Befindlichkeiten erkennen und Fahrmanöver entsprechend bewerten. Dies hat den Vorteil, dass ungünstige Befindlichkeiten der Fahrzeuginsassen von der Steuereinheit für autonomes Fahren vermieden werden können.

Vorzugsweise handelt es sich bei den physiologischen Messwerten um Gesundheitsdaten / Biodaten, wie etwa eine Herzfrequenz, eine Hautleitfähigkeit, eine Sauerstoffsättigung im Blut, einen Blutdruck, eine Konzentration eines Stresshormons wie etwa eine Adrenalinkonzentration und dergleichen. Dabei können den physiologischen Messwerten unterschiedliche Gewichtungen beigemessen werden. Beispielsweise kann dem Messwert für die Herzfrequenz eine höhere Gewichtung beigemessen werden als dem Messwert für die Hautleitfähigkeit. Alternativ oder zusätzlich kann es sich bei den physiologischen Messwerten um Informationen handeln, die durch Bildanalyse oder Sprachanalyse gewonnen werden. So kann beispielsweise eine Bilderkennung auf Grundlage von Aufnahmen einer Fahrzeuginnenraumkamera durchgeführt werden. Die Bilderkennung kann beispielsweise auf Gestenerkennung basieren. Beispielsweise kann durch Gestenerkennung festgestellt werden, wenn ein Fahrzeuginsasse eine Geste der Verärgerung ausführt oder die Augen über einen längeren Zeitraum schließt, und es können Rückschlüsse auf einen Angstzustand oder einen Stresszustand gezogen werden. Innenraumkameras können somit Gestiken analysieren und das Befinden der Fahrzeuginsassen bestimmen. Alternativ können anhand von Hautfarbe, Körperform (Schwangerschaftsbauch), Blut und dergleichen Gesundheitszustände bzw. Krankheiten ermittelt werden. Beispielsweise können durch Ermittlung von Helligkeitsunterschieden des von der Haut im Zeitverlauf reflektierten Lichts Vitalparameter, insbesondere die Pulsfrequenz, die Atemfrequenz und das Atemzugvolumen, ermittelt werden. Dieses Verfahren wird auch Photoplethysmographie genannt. Die Grundlagen des Verfahrens sind beispielsweise in dem Artikel von Ming-Zher Poh, Daniel J. McDuff, and Rosalind W. Picard, "Non-contact, automated cardiac pulse measurements using video imaging and blind source separation," Opt. Express 18, 10762-10774 (2010) beschrieben.

Es können auch Gespräche im Auto analysiert werden, um somit an Aussagen über die Fahrweise zu gelangen, die Rückschlüsse auf das Befinden eines Fahrzeuginsassen ermöglichen.

Die physiologischen Messwerte können beispielsweise von ein oder mehreren Benutzergeräten wie etwa einer Smartwatch erfasst werden und an die Steuereinheit für autonomes Fahren übermittelt werden. Durch Smartwatch und Fitnessarmbänder wird die Herzfrequenz ausgewertet und weitere Daten gesammelt und abgeleitet. Benutzergeräte wie eine Smartwatch, ein Mobiltelefon und andere Geräte können Daten sammeln und Aussagen über die Befindlichkeit und/oder Gesundheit der Fahrzeuginsassen ermöglichen. Alternativ können die physiologischen Messwerte von eingebauten Sensoren erfasst werden, wie beispielsweise von kapazitiven Sensoren, die in den Fahrersitz, die Kopfstütze, Armlehnen, Sicherheitsgurt oder dergleichen integriert sind und Herzfrequenz, Atmung oder Hirnströme nachweisen können, oder von Elektroden auf dem Lenkrad zum Nachweis der Herzfrequenz. Ebenso denkbar ist der Einsatz von Neuro-Headsets, welche die Gehirnaktivität und Emotionen registrieren, von Brustpulsgurten, implantierten Chips oder dergleichen. Die physiologischen Messwerte bzw. Gesundheitsdaten werden auf diese Weise von der Steuereinheit für autonomes Fahren vom Benutzergerät empfangen. Alternativ kann das Benutzergerät die physiologischen Messwerte bzw. Gesundheitsdaten auch selbst analysieren oder vorbearbeiten. Weiter alternativ können die Gesundheitsdaten auch von einer Cloud analysiert und aufbereitet und dann zurück ans Fahrzeug übermittelt werden (in Echtzeit oder im Postprocessing).
Vorzugsweise werden die physiologischen Messwerte zu einer Zeit ermittelt, die mit der Durchführung des Fahrmanövers korreliert. Beispielsweise werden die physiologischen Messwerte während der Durchführung des Fahrmanövers oder kurz nach Ausführung des Fahrmanövers ermittelt. Die physiologischen Messwerte können kontinuierlich ermittelt werden und beispielsweise mit entsprechenden Fahrmanövern in Bezug gesetzt werden.

Der Prozessor kann ferner dazu ausgelegt sein, auf Grundlage von bewerteten Fahrmanövern ein Benutzerprofil zu erstellen. In dem Benutzerprofil können beispiels-weise personenspezifische Bewertungen von vordefinierten Fahrmanövern bzw. Fahrmanövervarianten abgelegt werden.

Der Prozessor kann ferner dazu ausgelegt sein, ein anstehendes Fahrmanöver auf Grundlage des erstellten Benutzerprofils zu bewerten. Beispielsweise kann der Prozessor dazu ausgelegt sein, mehrere Varianten von Fahrmanövern auf Grundlage von gespeicherten Bewertungen in einem Benutzerprofil zu bewerten und eine optimale Variante auf Grundlage der Bewertung auszuwählen.

Das Benutzerprofil kann auch weitere Informationen umfassen. Beispielsweise besteht in aktuellen Fahrzeugen die Möglichkeit die Fahrwerkskomponenten einzustellen, beispielsweise das Fahrwerk straff/sportlich, komfortabel, ... einzustellen.
Ein sportliches Benutzerprofil hat beispielsweise sportlich eingestellte Fahrwerkskomponenten und spürt Bodenwellen. Ein ökonomisches Benutzerprofil hat komfortabel eingestellte Fahrwerkskomponenten und spürt die selben Bodenwellen weniger. Solche Einstellungen im Benutzerprofil können in die Bewertung von Fahrmanövern eingehen, beispielsweise in dem die den Fahrmanövervarianten zugeordneten Bewertungen entsprechend gewichtet werden. So können beispielsweise bei Einstellung eines sportlichen Profils Kurvenfahrten mit hohen Querbeschleunigungen besser bewertet werden, als bei einem ökonomisch eingestellten Benutzerprofil, falls dieses Anpassverhalten vom Hersteller oder Insassen gewünscht ist.

Benutzerprofile können abhängig von bestimmten Tagen und/oder Uhrzeiten sein oder abhängig vom Ziel (Bsp. morgens gemütlich, Nachmittag schnell, Wochenende komfortabel zeitoptimiert, ...).
Die Steuereinheit für autonomes Fahren kann dabei eine Steuereinheit für ein Lenksystem, eine Steuereinheit für ein Bremssystem und/oder eine Steuereinheit für einen Antriebstrang gemäß der ausgewählten Fahrmanövervariante ansteuern, so dass das ausgewählte Fahrmanöver bzw. die ausgewählte Fahrmanövervariante ausgeführt wird.

Der Prozessor kann ferner dazu ausgelegt sein, relative Veränderungen der physiologischen Messwerte zu erkennen und ein Fahrmanöver auf Grundlage der relativen Veränderungen der physiologischen Messwerte zu bewerten.

Der Prozessor kann ferner dazu ausgelegt sein, zu prüfen, ob eine Veränderungen der physiologischen Messwerte auf ein Fahrmanöver oder einen anderen Grund zurückzuführen ist und eine Bewertung des Fahrmanövers nur dann vorzunehmen, wenn ein anderer Grund auszuschließen ist. Beispielsweise könnte durch Zugriff auf eine Kalenderapplikation ein anderer Grund für die physiologischen Messwerte erkannt werden. Erkennt beispielsweise die Steuereinheit einen anstehenden Besprechungstermin im Kalender, und der Ort der Besprechung entspricht dem Fahrziel des Fahrzeugs oder dem Arbeitsort des Insassen, so könnte die Steuereinheit diese Information bei der Bewertung von Fahrmanövern mitberücksichtigen.
So kann beispielsweise davon ausgegangen werden, dass der Puls des Fahrers steigt je näher der Termin rückt, so dass der Pulsanstieg nicht mehr alleine auf das Fahrmanöver zurückzuführen ist und das Fahrmanöver deshalb nicht bewertet wird oder der erkannte Termindruck in die Bewertung mit einfließt.

Alternativ könnte die Steuereinheit auch so eingerichtet sein, dass sie, soweit sie Termindruck erkennt, die Fahrweise sportlicher gestaltet, so dass dem Insassen der Eindruck vermittelt wird, dass er schneller zum Ziel kommt.
Die Erfindung betrifft auch ein Verfahren für autonomes Fahren, bei dem ein durchgeführtes oder ein anstehendes Fahrmanöver auf Grundlage von physiologischen Messwerten bewertet wird. Bei dem Verfahren kann es sich um ein computerimplementiertes Verfahren handeln.

Ausführungsformen werden nun beispielhaft und unter Bezugnahme auf die beigefügten Zeichnungen beschrieben, in denen:
Fig. 1 ein Gesamtschema eines Ausführungsbeispiels eines Fahrzeugsteuerungssystems zeigt,
Fig. 2 ein Blockdiagramm zeigt, das schematisch die Konfiguration eines autonomen Fahrzeugs gemäß einem Ausführungsbeispiel der vorliegenden Erfindung darstellt,
Fig. 3 ein Blockdiagramm ist, das eine beispielhafte Konfiguration eines tragbaren Benutzergeräts zeigt,
Fig. 4 ein Blockdiagramm ist, das eine beispielhafte Konfiguration eines Steuergeräts für autonomes Fahren zeigt,
Fig. 5 vordefinierte Bewertungsgrößen zeigt, die gemäß einem Ausführungsbeispiel der Erfindung bestimmten Befindlichkeitszuständen des Fahrzeuginsassen zugeordnet sind,
Fig. 6a zeigt, wie gemäß einem Ausführungsbeispiel der Erfindung verschiedene Bereiche einer gemessenen Herzfrequenz vordefinierten Bewertungsgrößen zugeordnet werden,
Fig. 6b zeigt, wie gemäß einem alternativen Ausführungsbeispiel der Erfindung verschiedene Bereiche einer gemessenen Herzfrequenz kombiniert mit einer gemessenen Hautleitfähigkeit vordefinierten Bewertungsgrößen zugeordnet werden,
Fig. 6c zeigt, wie gemäß einem weiteren alternativen Ausführungsbeispiel der Erfindung verschiedene Bereiche einer Herzfrequenz auf benutzerspezifische Weise Bewertungsgrößen zugeordnet werden,
Fig. 6d zeigt, wie gemäß einem noch weiteren alternativen Ausführungsbeispiel der Erfindung verschiedene Bereiche einer Herzfrequenz Fahrmodus-spezifisch Bewertungsgrößen zugeordnet werden,
Fig. 7a eine schematische Darstellung mehrerer vordefinierter Fahrmanövervarianten für ein Fahrmanöver "Fahrt durch eine Kurve" zeigt,
Fig. 7b eine schematische Darstellung mehrerer vordefinierter Fahrmanövervarianten für ein Fahrmanöver "Fahrt auf der Autobahn" zeigt,
Fig. 8 eine beispielhafte Tabelle zeigt, die eine zeitliche Abfolge von vordefinierten und bewerteten Fahrmanövern umfasst,
Fig. 9 eine beispielhafte graphische Darstellung der von der Steuereinheit für autonomes Fahren ermittelten Bewertungsgrößen für die zeitliche Abfolge der Fahrmanöver aus der Fig. 8 zeigt,
Fig. 10 ein Flussdiagramm zeigt, das ein Verfahren für die Erstellung eines Benutzerprofils durch die Steuereinheit für autonomes Fahren veranschaulicht, bei dem die physiologischen Messwerte absolut bewertet werden,
Fig. 11 ein Flussdiagramm zeigt, das ein Verfahren für die Erstellung eines Benutzerprofils durch die Steuereinheit für autonomes Fahren veranschaulicht, bei dem die physiologischen Messwerte relativ (auf Veränderungen basierend) bewertet werden,
Fig. 12 eine beispielhafte Tabelle zeigt, die ein Benutzerprofil für die Fahrmanövervarianten eines Fahrmanövers "Fahrt durch eine Kurve" darstellt, und
FIG. 13 ein Flussdiagramm zeigt, das eine Bewertung von möglichen Fahrmanövervarianten eines anstehenden Fahrmanövers auf Grundlage eines Benutzerprofils veranschaulicht.

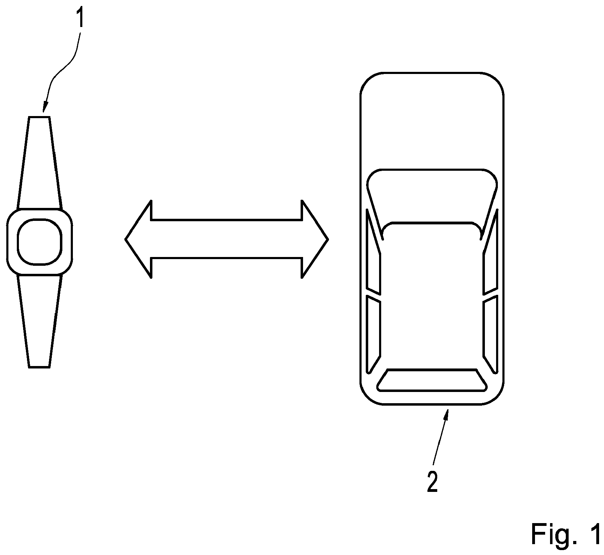

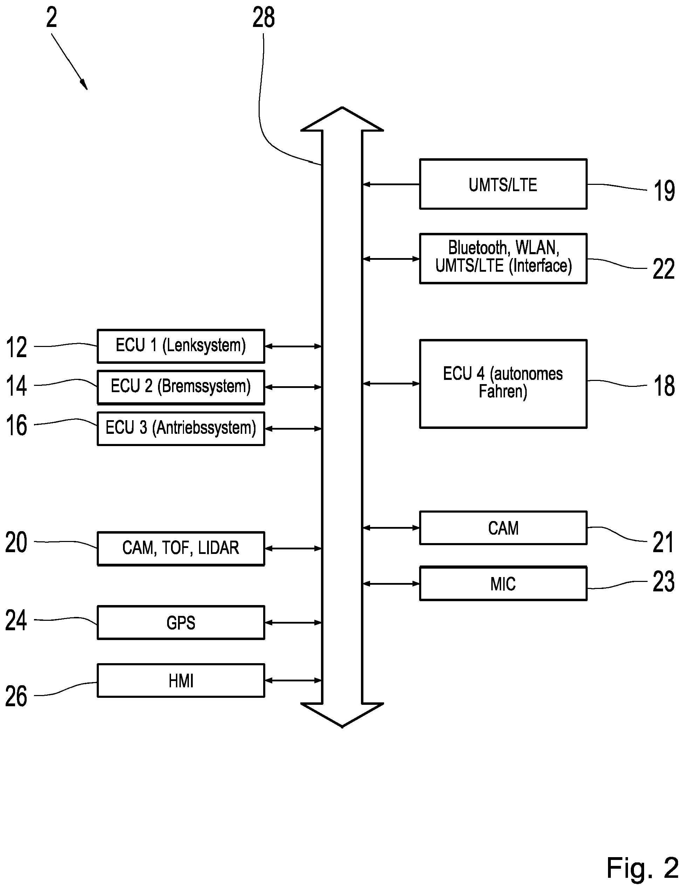

Fig. 1 zeigt ein Gesamtschema eines Ausführungsbeispiels eines Fahrzeugsteuerungssystems. Ein tragbares Benutzergerät 1, in diesem Fall eine Smartwatch, dient als eine Sensorvorrichtung, die physiologische Messwerte eines Fahrzeuginsassen bereitstellt. Das tragbare Benutzergerät 1 wird von einem Fahrzeuginsassen beispielsweise am Handgelenk oder an einer beliebigen anderen geeigneten Körperstelle getragen. Das tragbare Benutzergerät 1 kommuniziert mit einem autonomen (oder teilautonomen) Fahrzeug 2. Das tragbare Benutzergerät 1 ist dazu ausgelegt, einen oder mehrere physiologische Messwerte bezüglich des Fahrzeuginsassen zu ermitteln und über eine drahtlose Schnittstelle oder eine drahtgebundene Schnittstelle an eine Steuereinheit 18 für autonomes Fahren (siehe Fig. 2) im autonomen Fahrzeug 2 zu übertragen, so dass die Steuereinheit 18 für autonomes Fahren die erfassten Daten dazu nutzen kann, ein von dem Fahrzeug 2 zum Zeitpunkt der Erfassung der physiologischen Messwerte durchgeführtes Fahrmanöver auf Grundlage der erfassten physiologischen Messwerte des Fahrzeuginsassen zu bewerten.

Fig. 2 zeigt ein Blockdiagramm, das schematisch die Konfiguration eines Fahrzeugs 2 mit einer Steuereinheit für autonomes Fahren gemäß einem Ausführungsbeispiel der vorliegenden Erfindung darstellt. Das autonome Fahrzeug 2 umfasst mehrere elektronische Komponenten, welche via einem Fahrzeugkommunikationsnetzwerk 28 miteinander verbunden sind. Das Fahrzeugkommunikationsnetzwerk 28 kann beispielsweise ein im Fahrzeug eingebautes standardgemäßes Fahrzeugkommunikationsnetzwerk wie etwa ein CAN-Bus (controller area network), ein LIN-Bus (local interconnect network), ein LAN-Bus (local area network), ein MOST-Bus, ein FlexRay-Bus (registered trademark) und/oder ein Ethernet-Bussystem oder dergleichen sein.

In dem in Fig. 2 dargestellten Beispiel umfasst das autonome Fahrzeug 2 eine Steuereinheit 12 (ECU 1). Diese Steuereinheit 12 steuert ein Lenksystem. Das Lenksystem bezieht sich dabei auf die Komponenten, die eine Richtungssteuerung des Fahrzeugs ermöglichen.

Das autonome Fahrzeug 2 umfasst ferner eine Steuereinheit 14 (ECU 2), die ein Bremssystem steuert. Das Bremssystem bezieht sich dabei auf die Komponenten, die ein Bremsen des Fahrzeugs ermöglichen.

Das autonome Fahrzeug 2 umfasst ferner eine Steuereinheit 16 (ECU 3), die einen Antriebsstrang steuert. Der Antriebsstrang bezieht sich dabei auf die Antriebskomponenten des Fahrzeugs. Der Antriebsstrang kann einen Motor, ein Getriebe, eine Antriebs-/Propellerwelle, ein Differential und einen Achsantrieb umfassen.

Das autonome Fahrzeug 2 umfasst ferner eine Steuereinheit für autonomes Fahren 18 (ECU 4). Die Steuereinheit für autonomes Fahren 18 ist dazu ausgelegt, das autonome Fahrzeug 2 so zu steuern, dass dieses ganz oder teilweise ohne Einfluss eines menschlichen Fahrers fahren, steuern und einparken kann.

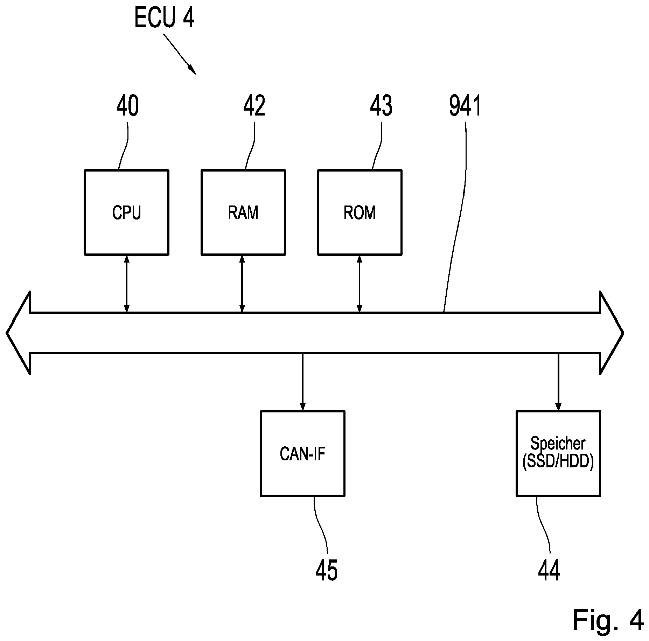

Die Steuereinheit für autonomes Fahren 18, die in Fig. 4 und der zugehörigen Beschreibung näher beschrieben ist, steuert ein oder mehrere Fahrzeugsubsysteme, während das Fahrzeug im autonomen Modus betrieben wird, nämlich das Bremssystem 14, das Lenksystem 12 und das Antriebssystem 16. Hierfür kann die Steuereinheit für autonomes Fahren 18 beispielsweise über das Fahrzeugkommunikationsnetzwerk 28 mit den entsprechenden Steuereinheiten 12, 14 und 16 kommunizieren. Die Steuereinheiten 12, 14 und 16 können ferner von den oben genannten Fahrzeugsubsystemen Fahrzeugbetriebsparameter empfangen, die diese mittels einem oder mehreren Fahrzeugsensoren erfassen. Fahrzeugsensoren sind vorzugsweise solche Sensoren, die einen Zustand des Fahrzeugs oder einen Zustand von Fahrzeugteilen erfassen, insbesondere deren Bewegungszustand. Die Sensoren können einen Fahrzeuggeschwindigkeitssensor, einen Gierraten-Sensor, einen Beschleunigungssensor, einen Lenkradwinkelsensor, einen Fahrzeuglastsensor, Temperatursensoren, Drucksensoren und dergleichen umfassen. Beispielsweise können auch Sensoren entlang der Bremsleitung angeordnet sein, um Signale auszugeben, die den Bremsflüssigkeitsdruck an verschiedenen Stellen entlang der hydraulischen Bremsleitung anzeigen. Andere Sensoren in der Nähe des Rades können vorgesehen sein, welche die Radgeschwindigkeit und den Bremsdruck erfassen, der am Rad aufgebracht wird.

Die Fahrzeugsensorik des autonomen Fahrzeugs 2 umfasst ferner eine Satellitennavigationseinheit 24 (GPS-Einheit). Es sei darauf hingewiesen, dass im Kontext der vorliegenden Erfindung GPS stellvertretend für sämtliche Globale Navigationssatellitensysteme (GNSS) steht, wie z.B. GPS, A-GPS, Galileo, GLONASS (Russland), Compass (China), IRNSS (Indien) und dergleichen.

Wenn steuerungsseitig oder fahrerseitig ein Betriebszustand für das autonome Fahren aktiviert ist, bestimmt die Steuereinheit 18 für autonomes Fahren, auf Grundlage von zur Verfügung stehenden Daten über eine vorgegebene Fahrtstrecke sowie von mittels den Fahrzeugsensoren erfassten Fahrzeugbetriebsparametern, die der Steuereinheit 18 von den Steuereinheiten 12, 14 und 16 zugeleitet werden, Parameter für den autonomen Betrieb des Fahrzeugs (beispielsweise Soll-Geschwindigkeit, Soll-Moment, Abstand zum Vordermann, Lenkvorgang und dergleichen).

Das autonome Fahrzeug 2 umfasst ferner einen oder mehrere Umfeldsensoren 20, die dazu ausgelegt sind, das Umfeld des Fahrzeugs zu erfassen, wobei die Umfeldsensoren 20 am Fahrzeug montiert sind und autark, d. h. ohne Informationssignale von außen, Objekte oder Zustände im Umfeld des Fahrzeugs erfassen. Hierzu zählen insbesondere Kameras, Radar-Sensoren, Lidar-Sensoren, Ultraschall-Sensoren oder dergleichen. Die Umfeldsensoren 20 können innerhalb des Fahrzeugs oder außerhalb des Fahrzeugs (z. B. an der Außenseite des Fahrzeugs) angeordnet sein können.

Das autonome Fahrzeug 2 umfasst ferner eine oder mehrere Innenraumkameras 21, die Bilddaten von Insassen des Fahrzeugs 2 liefern. Auf Grundlage der Bilddaten der Fahrzeuginsassen kann die Steuereinheit für autonomes Fahren 18 weitere Informationen über die Befindlichkeit der Fahrzeuginsassen erlangen und diese Kenntnis in die Bewertung von Fahrmanövern einfließen lassen, um eine Anpassung der Fahrweise an die Befindlichkeit bzw. an ein Profil der Insassen vorzunehmen. Wird beispielsweise festgestellt, dass ein Fahrzeuginsasse eine Geste der Verärgerung ausführt oder die Augen über einen längeren Zeitraum schließt, so können Rückschlüsse auf einen Angstzustand oder einen Stresszustand gezogen werden. Aus den Bilddaten können mit dem Fachmann bekannten Prozessen beispielsweise die Hauttemperatur, die Sauerstoffsättigung und der Blutdruck eines Fahrzeuginsassen ermittelt werden.

Das autonome Fahrzeug 2 umfasst ferner eine Benutzerschnittstelle 26 (HMI = Human-Machine-Interface), die es einem Fahrzeuginsassen ermöglicht, mit einem oder mehreren Fahrzeugsystemen in Interaktion zu stehen. Diese Benutzerschnittstelle 26 kann beispielsweise eine elektronische Anzeige (beispielsweise eine GUI = graphical user interface) zum Ausgeben einer Graphik, von Symbolen und/oder Inhalt in Textform, und eine Eingabeschnittstelle zum Empfangen einer Eingabe (beispielsweise manuelle Eingabe, Spracheingabe und Eingabe durch Gesten, Kopf- oder Augenbewegungen) umfassen. Die Eingabeschnittstelle kann beispielsweise Tastaturen, Schalter, berührungsempfindliche Bildschirme (Touchscreen), Eye-Tracker und dergleichen umfassen. Beispielsweise kann vom Benutzer über die Benutzerschnittstelle 26 ein persönliches Benutzerprofil eingestellt werden. Die Auswahl kann beispielsweise über ein Dropdown-Menü erfolgen, mithilfe dessen der Benutzer ein Benutzerprofil aus einer vorgegebenen Liste von Benutzerprofilen über Antippen eines Touchscreens oder durch Drücken von Tasten auf der Benutzerschnittstelle auswählen kann. Der Benutzer kann auch eine Benutzerauthentifikationsinformation an einen Server oder eine Cloud übertragen, auf dem/der mehrere Benutzerprofile bereitgestellt werden. In Abhängigkeit von der Benutzerauthentifikationsinformation können die für das Fahrzeug relevanten Konfigurationsdaten aus dem Benutzerprofil ausgewählt und über eine Kommunikationsverbindung an das Fahrzeug übertragen werden.

Das autonome Fahrzeug 2 umfasst ferner ein Mikrofon 23, sowie Mittel zur Spracherkennung und Sprachanalyse. Eine Sprachanalyse während der Fahrt erlaubt ebenfalls Rückschlüsse auf die Befindlichkeit der Fahrzeuginsassen.

Das autonome Fahrzeug 2 umfasst ferner eine Kommunikationsschnittstelle 19 für Mobilfunknetze. Diese umfasst beispielsweise eine Sim-Karte, mit der die Steuereinheit 18 über ein Mobilfunknetz wie UMTS oder LTE kommunizieren kann. Die Kommunikationsschnittstelle 19 kann beispielsweise die Kommunikation mit einer Cloud ermöglichen.

Das autonome Fahrzeug 2 umfasst ferner eine Kommunikationsschnittstelle 22 für externe Benutzergeräte, beispielsweise eine drahtlose WLAN oder Bluetooth-Schnittstelle, oder auch eine drahtgebundene USB-Verbindung. Die Kommunikationsschnittstelle 22 für externe Benutzergeräte dient beispielsweise zum Anbinden von Benutzergeräten wie Smartphones, Smartwatches und dergleichen.

Die Kommunikationsschnittstellen 22 und/oder 19 können auch Schnittstellen für den Austausch von Informationen und Daten zwischen Kraftfahrzeugen (C2C, Car-to-Car-Kommunikation) bzw. zwischen Fahrzeugen und einer Verkehrsinfrastruktur (C2X, X2C) bereitstellen.

Wie in den folgenden Ausführungsbeispielen detaillierter beschrieben ist, empfängt die Steuereinheit 18 für autonomes Fahren über die Kommunikationsschnittstelle 22 von einem tragbaren Benutzergerät (1 in Figur 1) kontinuierlich physiologische Messwerte bezüglich eines oder mehrerer Fahrzeuginsassen. Die Steuereinheit für autonomes Fahren 18 ist dazu ausgelegt, auf Basis dieser physiologischen Messwerte eine Bewertung von Fahrmanövern durchzuführen, die zu einem Zeitpunkt durchgeführt wurden, der mit dem Zeitpunkt der Erfassung der physiologischen Messwerte durch das tragbare Benutzergerät 1 korreliert.

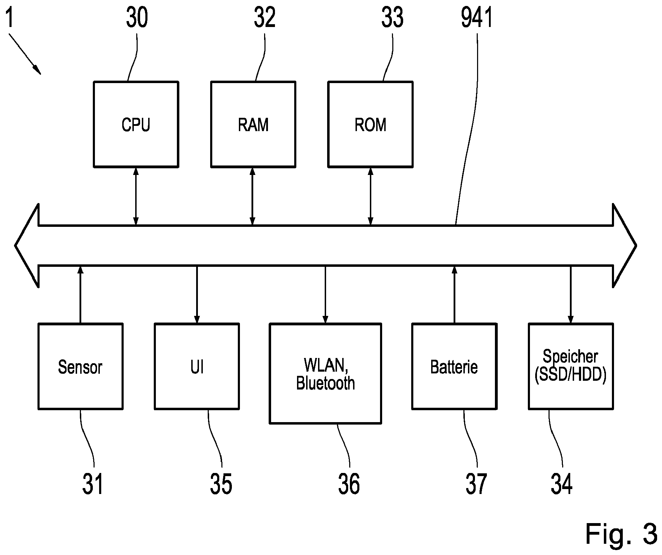

Fig. 3 zeigt ein Blockdiagramm, das eine beispielhafte Konfiguration eines tragbaren Benutzergeräts 1 darstellt, wobei das Benutzergerät dazu ausgelegt ist, physiologische Messwerte bereitzustellen. Das tragbare Benutzergerät 1 umfasst einen Prozessor 30. Bei dem Prozessor 30 kann es sich beispielsweise um eine Recheneinheit wie eine zentrale Verarbeitungseinheit (CPU = central processing unit) handeln, die Programminstruktionen ausführt. Das tragbare Benutzergerät 1 umfasst ferner Speicherkomponenten, beispielsweise einen Direktzugriffsspeicher 32 (RAM = Random Access Memory) oder einen Nur-Lese-Speicher 33 (ROM = Read-only memory) und Eingabe-/Ausgabe-schaltungen, die funktionell damit verbunden sind. Der RAM-Speicher 32 speichert die vom Prozessor 30 benutzten Programme, um verschiedene Parameter zu bestimmen. In dem ROM-Speicher 33 mit direktem Zugriff werden die zum Ausführen der Programme notwendigen Informationen und aktuellen Daten ausgelesen, die in diesen eingeschrieben sind. Ferner ist es ebenso möglich, ein externes Speicherlaufwerk 34, wie beispielsweise ein externes Festplattenlaufwerk (hard disk drive: HDD), ein Flashspeicher-Laufwerk oder ein nicht flüchtiges Festkörperlaufwerk (solid state drive: SSD), anzuschließen.

Das tragbare Benutzergerät 1 umfasst ferner einen oder mehrere Biosensoren 31, die dazu ausgelegt sind, physiologische Messwerte bezüglich eines Fahrzeuginsassen zu erfassen. Diese umfassen bevorzugt einen oder mehrere der folgenden Sensoren: einen Sensor zum Ermitteln der Herzfrequenz und der Blutsauerstoffsättigung, wobei dieser Sensor vorzugsweise durch einen optischen Sensor (beispielsweise einen Photoplethysmographie-Sensor) gebildet wird; einen Sensor zur Messung der elektrischen Leitfähigkeit der Haut, insbesondere der elektrodermalen Aktivität; einen Sensor zur Messung der Temperatur oder eines Wärmestroms, einen Sensor zur Messung der Atmung, einen Sensor zur Überwachung des Blutdrucks, einen Sensor zur Überwachung des Muskeltonus und dergleichen.

Die Sensoren können in einem einzigen tragbaren Benutzergerät 1 angeordnet sein, welches beispielsweise als Armband, als Fingerclip oder am Ohr getragen werden kann. Alternativ können auch mehrere Benutzergeräte separat physiologische Messwerte an die Steuereinheit für autonomes Fahren bereitstellen.

Das tragbare Benutzergerät 1 umfasst eine Kommunikationsschnittstelle 36, beispielsweise einen Funksender, mit dem die erfassten Daten drahtlos, insbesondere über UMTS, WLAN oder Bluetooth, über die Kommunikationsschnittstelle 22 und das Fahrzeugkommunikationsnetzwerk 28 des autonomen Fahrzeugs 2 an die Steuereinheit für autonomes Fahren 18 (siehe Fig. 4) übertragen werden können.

Ferner umfasst das tragbare Benutzergerät 1 eine Energiequelle 37. Die Energiequelle 37 ist vorzugsweise in Form einer Batterie ausgeführt und versorgt die angeschlossenen Bauteile mit elektrischer Energie. Ferner verfügt tragbare Benutzergerät 1 über eine Benutzerschnittstelle 35 (User Interface Ul), mittels derer der Nutzer Informationen ablesen oder Eingaben eingeben kann. Diese Benutzerschnittstelle 35 kann beispielsweise eine Anzeige umfassen, insbesondere eine LED-Anzeige, die dem Träger des tragbaren Benutzergeräts 1 einen gemessenen physiologischen Zustand signalisieren kann.

Fig. 4 zeigt ein Blockdiagramm, das eine beispielhafte Konfiguration einer Steuereinheit für autonomes Fahren 18 (ECU 4) darstellt. Bei der Steuereinheit für autonomes Fahren 18 kann es sich beispielsweise um ein Steuergerät (electronic control unit ECU oder electronic control module ECM) handeln. Die Steuereinheit für autonomes Fahren 18 (ECU 4) umfasst einen Prozessor 40. Bei dem Prozessor 40 kann es sich beispielsweise um eine Recheneinheit wie eine zentrale Verarbeitungseinheit (CPU = central processing unit) handeln, die Programminstruktionen ausführt.

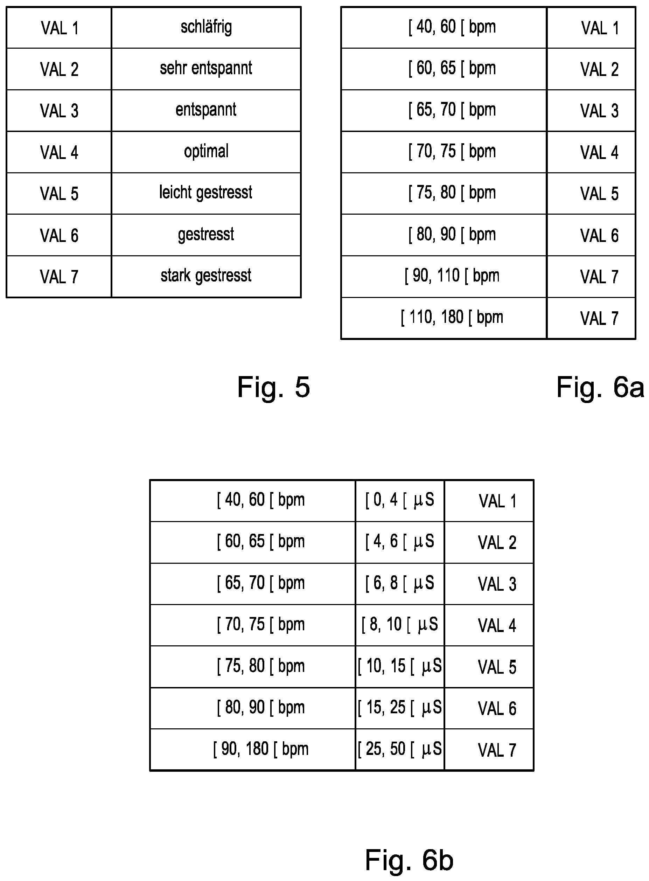

Der Prozessor der Steuereinheit für autonomes Fahren 18 ist dazu ausgelegt ist, ein durchgeführtes oder ein anstehendes Fahrmanöver zu bewerten, indem diesem eine Bewertung zugeordnet wird. Die Bewertung kann beispielsweise durch einen Parameter, einen Zahlenwert, oder dergleichen definiert sein. Beispielsweise können verschiedene Bewertungsgrößen in der Steuereinheit vordefiniert sein, wobei jede Bewertungsgröße einer Befindlichkeit eines Fahrzeuginsassen zugeordnet ist (siehe Fig. 5).

Die Steuereinheit für autonomes Fahren 18 umfasst ferner einen Speicher und eine Eingabe/ Ausgabe-Schnittstelle. Der Speicher kann aus einem oder mehreren nichtflüchtigen computerlesbaren Medien bestehen und umfasst mindestens einen Programmspeicherbereich und einen Datenspeicherbereich. Der Programmspeicherbereich und der Datenspeicherbereich können Kombinationen von verschiedenen Arten von Speicher umfassen, beispielsweise von einem Nur-Lese-Speicher 43 (ROM = Read-only memory) und einem Direktzugriffsspeicher 42 (RAM = Random Access Memory) (z. B. dynamischer RAM ("DRAM"), synchron DRAM ("SDRAM") usw.). Ferner kann die Steuereinheit für autonomes Fahren 18 ein externes Speicherlaufwerk 44, wie beispielsweise ein externes Festplattenlaufwerk (hard disk drive: HDD), ein Flashspeicher-Laufwerk oder ein nicht flüchtiges Festkörperlaufwerk (solid state drive: SSD) umfassen.

Die Steuereinheit für autonomes Fahren 18 umfasst ferner eine Kommunikationsschnittstelle 45, über welche die Steuereinheit mit dem Fahrzeugkommunikationsnetzwerk (28 in Fig. 2) kommunizieren kann.

FIG. 5 zeigt vordefinierte Bewertungsgrößen VAL1-VAL7, die gemäß einem Ausführungsbeispiel der Erfindung bestimmten Befindlichkeitszuständen des Fahrzeuginsassen zugeordnet sind. Die Bewertungsgrößen VAL1-VAL7 sind beispielsweise im Speicher (44 in Fig. 4) der Steuereinheit (18 in Fig. 2) für autonomes Fahren hinterlegt. Eine vordefinierte Bewertungsgröße VAL1 ist einem Befindlichkeitszustand "schläfrig" zugeordnet. Eine vordefinierte Bewertungsgröße VAL2 ist einem Befindlichkeitszustand "sehr entspannt" zugeordnet. Eine vordefinierte Bewertungsgröße VAL3 ist einem Befindlichkeitszustand "entspannt" zugeordnet. Eine vordefinierte Bewertungsgröße VAL4 ist einem Befindlichkeitszustand "optimal" zugeordnet. Eine vordefinierte Bewertungsgröße VAL5 ist einem Befindlichkeitszustand "leicht gestresst" zugeordnet. Eine vordefinierte Bewertungsgröße VAL6 ist einem Befindlichkeitszustand "gestresst" zugeordnet. Eine vordefinierte Bewertungsgröße VAL7 ist einem Befindlichkeitszustand "stark gestresst" zugeordnet. Durch Definition dieser Bewertungsgrößen können Befindlichkeitszustände wie Stress, Ärger, Schläfrigkeit, Unterforderung oder dergleichen quantifiziert und, wie im Folgenden genauer beschrieben, Fahrmanövern zugeordnet werden. Sollte das Fahrzeug beispielsweise sehr ökonomisch und komfortabel unterwegs sein, die Insassen es aber eilig haben, so dass sie genervt von der "ruhigen" Fahrweise sind, so kann das Fahrzeug anhand von physiologischen Messwerten langsame Fahrmanöver mit Bewertungsgrößen VAL1 bis VAL3 als nicht optimal bewerten und damit die Fahrweise sportlicher gestalten.

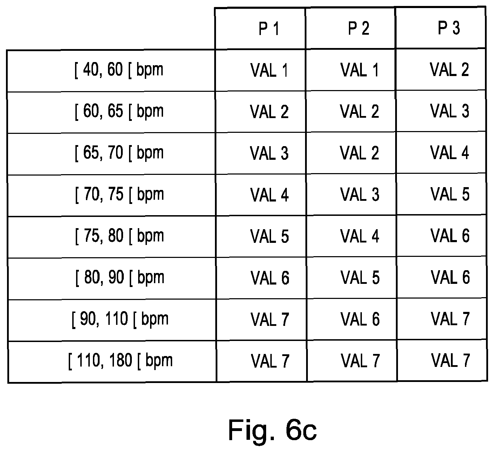

Fig. 6a zeigt, wie gemäß einem Ausführungsbeispiel der Erfindung verschiedene Bereiche einer Herzfrequenz, die von dem tragbaren Benutzergerät 1 erfasst wird, den oben genannten vordefinierten Bewertungsgrößen VAL1-VAL7 zugeordnet werden. Einer Herzfrequenz zwischen 40 und 60 Schlägen pro Minute (beats per minute bpm) wird die Bewertungsgröße VAL1 zugeordnet; einer Herzfrequenz zwischen 60 und 65 bpm wird die Bewertungsgröße VAL2 zugeordnet; einer Herzfrequenz zwischen 65 und 70 bpm wird die Bewertungsgröße VAL3 zugeordnet; einer Herzfrequenz zwischen 70 und 75 bpm wird die Bewertungsgröße VAL4 zugeordnet; einer Herzfrequenz zwischen 75 und 80 bpm wird die Bewertungsgröße VAL5 zugeordnet; einer Herzfrequenz zwischen 80 und 90 bpm wird die Bewertungsgröße VAL6 zugeordnet; einer Herzfrequenz zwischen 90 und 110 bpm wird die Bewertungsgröße VAL7 zugeordnet und einer Herzfrequenz zwischen 110 und 180 bpm wird ebenfalls die Bewertungsgröße VAL7 zugeordnet. Auch diese Zuordnungen können im Voraus im Speicher (44 in Fig. 4) der Steuereinheit (18 in Fig. 2) für autonomes Fahren hinterlegt werden. Die Zuordnung kann benutzerspezifisch sein. Beispielsweise kann die Zuordnung vom Alter des Fahrzeuginsassen abhängig sein und in einem jeweiligen Benutzerprofil hinterlegt werden (siehe Ausführungsbeispiel der Fig. 6c).

Fig. 6a zeigt eine beispielhafte Zuordnung zu einer bestimmten Person. Die Zuordnung kann von der Steuereinheit für autonomes Fahren vordefiniert werden, oder von ihr dynamisch erstellt (gelernt) werden. Der Bewertungsalgorithmus kann evtl. zunächst die Normalparameter (z.B. einen Ruhepuls) bestimmen, um eine Zuordnung zu Befindenszuständen ermöglichen zu können.

Das Befinden eines Fahrzeuginsassen kann sich auch durch einen erhöhten Adrenalinspiegel, eine bestimmte Haltung, eine veränderte Hautleitfähigkeit, eine veränderte Sauerstoffsättigung im Blut oder dergleichen ausdrücken.

Es können auch mehrere Typen von Messgrößen erfasst werden und aus einer Zusammenschau aller ermittelten Messgrößen auf einen Befindlichkeitszustand des Fahrzeuginsassen rückgeschlossen werden. Beispielsweise können hierfür vordefinierte mehrdimensionale Tabellen hinterlegt werden, oder Bewertungsgrößen können analytisch durch Berechnung aus mehreren Messgrößen berechnet werden. Dabei können bei der Zuordnung der Bewertungsgrößen im Fall von mehreren Messgrößen den physiologischen Messwerten unterschiedliche Gewichtungen beigemessen werden. Beispielsweise kann dem Messwert für die Herzfrequenz eine höhere Gewichtung beigemessen werden als dem Messwert für die Hautleitfähigkeit.

Fig. 6b zeigt beispielhaft, wie gemäß einem alternativen Ausführungsbeispiel der Erfindung verschiedene Bereiche einer Herzfrequenz, die von dem tragbaren Benutzergerät 1 erfasst wird, kombiniert mit einer gemessenen Hautleitfähigkeit den oben genannten vordefinierten Bewertungsgrößen (VAL1-VAL7) zugeordnet werden. Beispielsweise wird einer Herzfrequenz zwischen 40 und 60 bpm kombiniert mit einer Hautleitfähigkeit zwischen 0 und 4 Mikrosiemens die Bewertungsgröße VAL1 zugeordnet; einer Herzfrequenz zwischen 60 und 65 bpm kombiniert mit einer Hautleitfähigkeit zwischen 4 und 6 Mikrosiemens wird die Bewertungsgröße VAL2 zugeordnet; einer Herzfrequenz zwischen 65 und 70 bpm kombiniert mit einer Hautleitfähigkeit zwischen 6 und 8 Mikrosiemens wird die Bewertungsgröße VAL3 zugeordnet usw.

Fig. 6c zeigt, wie gemäß einem weiteren alternativen Ausführungsbeispiel der Erfindung verschiedene Bereiche einer Herzfrequenz auf benutzerspezifische Weise Bewertungsgrößen zugeordnet werden. Einer Herzfrequenz zwischen 40 und 60 bpm wird für einen Benutzer P1 und einen Benutzer P2 die Bewertungsgröße VAL1 und für einen Benutzer P3 die Bewertungsgröße VAL2 zugeordnet; einer Herzfrequenz zwischen 60 und 65 bpm wird für den Benutzer P1 und den Benutzer P2 die Bewertungsgröße VAL2 und für den Benutzer P3 die Bewertungsgröße VAL3 zugeordnet; einer Herzfrequenz zwischen 65 und 70 bpm wird für den Benutzer P1 die Bewertungsgröße VAL3, für den Benutzer P2 die Bewertungsgröße VAL2 und für den Benutzer P3 die Bewertungsgröße VAL4 zugeordnet; einer Herzfrequenz zwischen 70 und 75 bpm wird für den Benutzer P1 die Bewertungsgröße VAL4, für den Benutzer P2 die Bewertungsgröße VAL3 und für den Benutzer P3 die Bewertungsgröße VAL5 zugeordnet; einer Herzfrequenz zwischen 75 und 80 bpm wird für den Benutzer P1 die Bewertungsgröße VAL5, für den Benutzer P2 die Bewertungsgröße VAL4 und für den Benutzer P3 die Bewertungsgröße VAL6 zugeordnet; einer Herzfrequenz zwischen 80 und 90 bpm wird für den Benutzer P1 die Bewertungsgröße VAL6, für den Benutzer P2 die Bewertungsgröße VAL5 und für den Benutzer P3 die Bewertungsgröße VAL6 zugeordnet; einer Herzfrequenz zwischen 90 und 110 bpm wird für den Benutzer P1 die Bewertungsgröße VAL7, für den Benutzer P2 die Bewertungsgröße VAL6 und für den Benutzer P3 die Bewertungsgröße VAL7 zugeordnet; und einer Herzfrequenz zwischen 110 und 180 bpm wird für die Benutzer P1, P2 und P3 die Bewertungsgröße VAL7 zugeordnet. Auch diese Zuordnungen können im Voraus im Speicher (44 in Fig. 4) der Steuereinheit (18 in Fig. 2) für autonomes Fahren hinterlegt werden. Beispielsweise kann die Zuordnung vom Alter des Fahrzeuginsassen abhängig sein und in einem jeweiligen Benutzerprofil hinterlegt werden. Alternativ kann diese Zuordnung auch von mehreren Parametern abhängen, wie beispielsweise Alter, Geschlecht, Körperfettanteil, Vorerkrankungen und dergleichen. Das Gleiche gilt für Gesten, Hautfarbe und alle anderen physiologischen Größen.

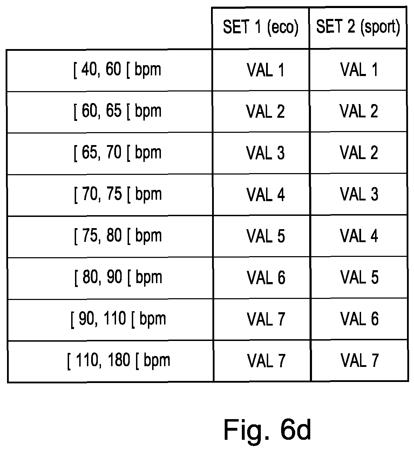

Fig. 6d zeigt, wie gemäß einem noch weiteren alternativen Ausführungsbeispiel der Erfindung verschiedene Bereiche einer Herzfrequenz Fahrmodus-spezifisch Bewertungsgrößen zugeordnet werden. Es werden zwei unterschiedliche Fahrmodi vorausgesetzt, ein erster Modus SET1, bei dem es sich um einen Fahrmodus mit ökonomischer Fahrweise handelt und ein zweiter Fahrmodus SET2, bei dem es sich um einen Fahrmodus mit sportlicher Fahrweise handelt. Der Fahrmodus kann beispielsweise von einem Fahrzeuginsassen mittels einer Benutzerschnittstelle eingestellt werden. Einer Herzfrequenz zwischen 40 und 60 bpm wird für einen Fahrmodus SET1 die Bewertungsgröße VAL1 und für einen Fahrmodus SET2 ebenfalls die Bewertungsgröße VAL1 zugeordnet; einer Herzfrequenz zwischen 60 und 65 bpm wird für den Fahrmodus SET1 die Bewertungsgröße VAL2 und für den Fahrmodus SET2 ebenfalls die Bewertungsgröße VAL2 zugeordnet; einer Herzfrequenz zwischen 65 und 70 bpm wird für den Fahrmodus SET1 die Bewertungsgröße VAL3 und für den Fahrmodus SET2 die Bewertungsgröße VAL2 zugeordnet; einer Herzfrequenz zwischen 70 und 75 bpm wird für den Fahrmodus SET1 die Bewertungsgröße VAL4 und für den Fahrmodus SET2 die Bewertungsgröße VAL3 zugeordnet; einer Herzfrequenz zwischen 75 und 80 bpm wird für den Fahrmodus SET1 die Bewertungsgröße VAL5 und für den Fahrmodus SET2 die Bewertungsgröße VAL4 zugeordnet; einer Herzfrequenz zwischen 80 und 90 bpm wird für den Fahrmodus SET1 die Bewertungsgröße VAL6 und für den Fahrmodus SET2 die Bewertungsgröße VAL5 zugeordnet; einer Herzfrequenz zwischen 90 und 110 bpm wird für den Fahrmodus SET1 die Bewertungsgröße VAL7 und für den Fahrmodus SET2 die Bewertungsgröße VAL6 zugeordnet; und einer Herzfrequenz zwischen 110 und 180 bpm wird für den Fahrmodus SET1 die Bewertungsgröße VAL7 und für den Fahrmodus SET2 ebenfalls die Bewertungsgröße VAL7 zugeordnet. Auch diese Zuordnungen können im Voraus im Speicher (44 in Fig. 4) der Steuereinheit (18 in Fig. 2) für autonomes Fahren hinterlegt werden. Der Tabelle ist zu entnehmen, dass im sportlichen Fahrmodus die optimale Bewertung bei einer höheren Herzfrequenz (hier [75, 80[ bpm) vorliegt, als im ökonomischen Fahrmodus (hier [70, 75[ bpm).

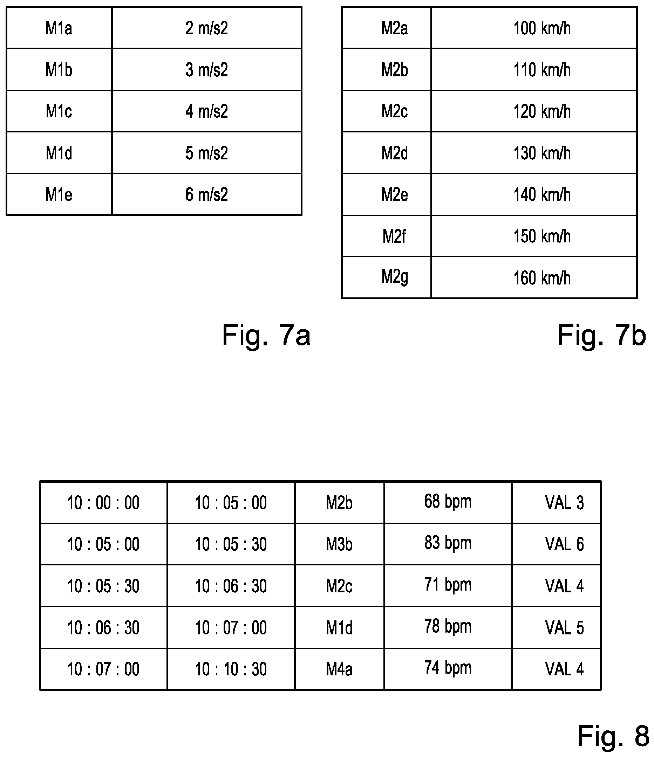

Fig. 7a zeigt gemäß einem Ausführungsbeispiel der Erfindung eine schematische Darstellung mehrerer vordefinierter Fahrmanövervarianten M1a bis M1e für ein Fahrmanöver M1. Im vorliegenden Ausführungsbeispiel entspricht das Fahrmanöver M1 einer Fahrt durch eine Kurve und die Fahrmanövervarianten M1a bis M1e entsprechen einer Fahrt durch eine Kurve mit einer vordefinierten Querbeschleunigung. Ein Fahrmanöver M1a entspricht einer Fahrt durch eine Kurve mit einer Querbeschleunigung von 2 m/s²; ein Fahrmanöver M1b entspricht einer Fahrt durch eine Kurve mit einer Querbeschleunigung von 3 m/s²; ein Fahrmanöver M1c entspricht einer Fahrt durch eine Kurve mit einer Querbeschleunigung von 4 m/s²; ein Fahrmanöver M1d entspricht einer Fahrt durch eine Kurve mit einer Querbeschleunigung von 5 m/s²; und ein Fahrmanöver M1e entspricht einer Fahrt durch eine Kurve mit einer Querbeschleunigung von 6 m/s².

Im obigen Beispiel sind die Fahrmanöver M1a - M1e alleine durch die Querbeschleunigung und den Kontext "Kurvenfahrt" festgelegt. In alternativen Ausführungsbeispielen können auch mehrere Fahrzeugbetriebsparameter, die von den Fahrzeugsensoren erfasst werden zur Definition eines Fahrmanövers herangezogen werden.

Fig. 7b zeigt eine schematische Darstellung mehrerer vordefinierter Fahrmanövervarianten M2a bis M2g für ein Fahrmanöver M2. Im vorliegenden Fall entspricht das Fahrmanöver M2 einer Fahrt auf der Autobahn und die Fahrmanövervarianten M2a bis M2g entsprechen einer Fahrt auf der Autobahn mit einer vordefinierten Geschwindigkeit. Ein Fahrmanöver M2a entspricht einer Fahrt auf der Autobahn mit einer
Geschwindigkeit von 100 km/h; ein Fahrmanöver M2b entspricht einer Fahrt auf der Autobahn mit einer Geschwindigkeit von 110 km/h; ein Fahrmanöver M2c entspricht einer Fahrt auf der Autobahn mit einer Geschwindigkeit von 120 km/h; ein Fahrmanöver M2d entspricht einer Fahrt auf der Autobahn mit einer Geschwindigkeit von 130 km/h; ein Fahrmanöver M2e entspricht einer Fahrt auf der Autobahn mit einer Geschwindigkeit von 140 km/h; ein Fahrmanöver M2f entspricht einer Fahrt auf der Autobahn mit einer Geschwindigkeit von 150 km/h; und ein Fahrmanöver M2g entspricht einer Fahrt auf der Autobahn mit einer Geschwindigkeit von 160 km/h.

Im obigen Beispiel sind die Fahrmanöver M2a - M2g alleine durch die Geschwindigkeit und den Kontext "Autobahnfahrt" festgelegt. In alternativen Ausführungsbeispielen können auch mehrere Fahrzeugbetriebsparameter, die von den Fahrzeugsensoren erfasst werden zur Definition eines Fahrmanövers herangezogen werden. Des Weiteren können auch die Verkehrsbedingungen berücksichtigt werden. So können beispielsweise umgebungsbezogene Zustandsparameter von anderen Fahrzeugen herangezogen werden, insbesondere deren Position, Geschwindigkeit oder Fahrtrichtung, Gefahrensituationen und dergleichen, die über fahrzeugbasierte Kommunikationssysteme (z. B. eine Car-to-X-Schnittstelle und/oder eine X-to-Car-Schnittstelle) übermittelt werden. Außerdem kann die Verkehrsdichte unter Einsatz mitgeführter Sensoren geschätzt werden, wie zum Beispiel Radar, Lidar und optische Kameras, die auf einem Fahrzeug bereits als Teil anderer Systeme verfügbar sein können.

Fig. 8 zeigt eine beispielhafte Tabelle, die eine zeitliche Abfolge von vordefinierten Fahrmanövern umfasst, die im Rahmen eines zurückgelegten Fahrtabschnitts durchgeführt werden. Die Tabelle repräsentiert das Ergebnis der Ermittlung physiologischer Messgrößen während des bestimmten Fahrtabschnitts, der hier von 10:00:00 bis 10:10:30 gedauert hat. Die Tabelle umfasst Messwerte für die Herzfrequenz, die von dem tragbaren Benutzergerät 1 zu Zeitpunkten gemessen werden, die mit den jeweiligen Fahrmanövern korrelieren. Das Fahrmanöver ist der Steuereinheit für autonomes Fahren aus dem Steuer- und Navigationskontext bekannt und wird auf dem Fachmann bekannte Weise ermittelt. Wie aus der Tabelle ersichtlich, erfolgt von dem Zeitpunkt t = 10:00:00 bis zu dem Zeitpunkt t = 10:05:00 ein Fahrmanöver M2b (Fahrt auf der Autobahn mit einer Geschwindigkeit von 110 km/h, siehe Fig. 7b) und die gemessene Herzfrequenz beträgt 68 bpm. Gemäß der vordefinierten Zuordnung (Fig. 6a) ist dieser Herzfrequenz die Bewertungsgröße VAL3 zugeordnet, die "entspannt" repräsentiert. Von dem Zeitpunkt t = 10:05:00 bis zu dem Zeitpunkt t = 10:05:30 erfolgt ein Fahrmanöver M3b (ein Überhohlvorgang) und die gemessene Herzfrequenz beträgt 83 bpm. Gemäß der vordefinierten Zuordnung (Fig. 6a) ist dieser Herzfrequenz die Bewertungsgröße VAL6 zugeordnet, die "gestresst" repräsentiert. Von dem Zeitpunkt t = 10:05:30 bis zu dem Zeitpunkt t = 10:06:30 erfolgt ein Fahrmanöver M2c (Fahrt auf der Autobahn mit einer Geschwindigkeit von 120 km/h, siehe Fig. 7b) und die gemessene Herzfrequenz beträgt 71 bpm. Gemäß der vordefinierten Zuordnung (Fig. 6a) ist dieser Herzfrequenz die Bewertungsgröße VAL4 zugeordnet, die "optimal" repräsentiert. Von dem Zeitpunkt t = 10:06:30 bis zu dem Zeitpunkt t = 10:07:00 erfolgt ein Fahrmanöver M1d (Fahrt durch eine Kurve mit einer Querbeschleunigung von 5 m/s², siehe Fig. 7a) und die gemessene Herzfrequenz beträgt 78 bpm. Gemäß der vordefinierten Zuordnung (Fig. 6a) ist dieser Herzfrequenz die Bewertungsgröße VAL5 zugeordnet, die "leicht gestresst" repräsentiert. Im vorliegenden Ausführungsbeispiel entspricht das Fahrmanöver M1d einer Fahrt durch eine Kurve mit einer Querbeschleunigung von 5 m/s², die bei der Abfahrt von der Autobahn erfolgt. Die Steuereinheit des autonomen Fahrzeugs hat die Aufgabe, die Autobahn an der nächsten Ausfahrt zu verlassen. Das Fahrzeug wechselt den Fahrstreifen nach rechts auf den Verzögerungsstreifen. Kurz vor Beginn der Kurve an der Ausfahrt wird das Fahrzeug stark verzögert. Die Querbeschleunigung in der Kurve ist durch das spät eingeleitete Verzögern relativ hoch, so dass sich ein Fahrzeuginsasse unwohl fühlt und dessen Herzfrequenz auf 78 bpm ansteigt. Von dem Zeitpunkt t = 10:07:00 bis zu dem Zeitpunkt t = 10:10:30 erfolgt ein Fahrmanöver M4a (eine Fahrt auf der Landstraße mit einer Geschwindigkeit von 70 km/h) und die gemessene Herzfrequenz beträgt 74 bpm. Gemäß der vordefinierten Zuordnung (Fig. 6a) ist dieser Herzfrequenz die Bewertungsgröße VAL4 zugeordnet, die "optimal" repräsentiert.

Der Messwert für die Herzfrequenz kann dabei auch ein Mittelwert aus mehreren Messwerten sein, die zeitgleich mit einem bestimmten Fahrmanöver ermittelt wurden.

Aus den ermittelten physiologischen Messwerten werden folglich von der Steuereinheit für autonomes Fahren auf Grundlage der vordefinierten Bewertungen die zugehörigen Bewertungsgrößen VAL1 bis VAL7 ermittelt, die emotionale Zustände wie Entspannung bzw. Stress oder Schläfrigkeit repräsentieren und damit die ausgeführten Fahrmanöver bewertet. Somit kann die Steuereinheit für autonomes Fahren 18 ausgehend von den erfassten physiologischen Messwerten ermitteln, ob sich der Fahrer in einem entspannten Zustand befindet oder, im Gegenteil, eher angespannt oder sogar gestresst ist.

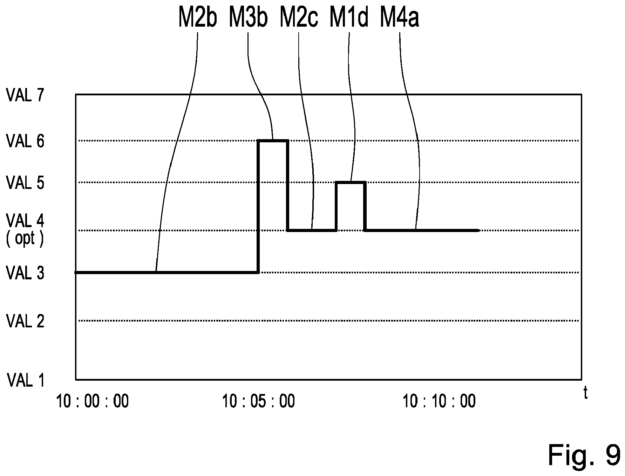

Fig. 9 zeigt eine graphische Darstellung der von der Steuereinheit für autonomes Fahren (18 in Fig. 2) ermittelten Bewertungsgrößen für die zeitliche Abfolge der Fahrmanöver aus der Fig. 8. Von dem Zeitpunkt t = 10:00:00 bis zu dem Zeitpunkt t = 10:05:00 wurde das Fahrmanöver M2b (Fahrt auf der Autobahn mit einer Geschwindigkeit von 110 km/h, siehe Fig. 7b) registriert, dem die Bewertungsgröße VAL3 zugeordnet ist, die "entspannt" repräsentiert. Von dem Zeitpunkt t = 10:05:00 bis zu dem Zeitpunkt t = 10:05:30 wurde ein Fahrmanöver M3b (ein Überhohlvorgang) registriert, dem die Bewertungsgröße VAL6 zugeordnet ist, die "gestresst" repräsentiert. Von dem Zeitpunkt t = 10:05:30 bis zu dem Zeitpunkt t = 10:06:30 wurde ein Fahrmanöver M2c (Fahrt auf der Autobahn mit einer Geschwindigkeit von 120 km/h, siehe Fig. 7b) registriert, dem die Bewertungsgröße VAL4 zugeordnet ist, die "optimal" repräsentiert. Von dem Zeitpunkt t = 10:06:30 bis zu dem Zeitpunkt t = 10:07:00 wurde ein Fahrmanöver M1d (Fahrt durch eine Kurve mit einer Querbeschleunigung von 5 m/s², siehe Fig. 7a) registriert, dem die Bewertungsgröße VAL5 zugeordnet ist, die "leicht gestresst" repräsentiert. Von dem Zeitpunkt t = 10:07:00 bis zu dem Zeitpunkt t = 10:10:30 wurde ein Fahrmanöver M4a (eine Fahrt auf der Landstraße mit einer Geschwindigkeit von 70 km/h) registriert dem die Bewertungsgröße VAL4 zugeordnet ist, die "optimal" repräsentiert.

Auf diese Weise kann beispielsweise kontinuierlich oder während spezieller Trainings- bzw. Lernphasen eine Bewertung der Fahrmanöver der Steuereinheit für autonomes Fahren auf Grundlage der gemessenen physiologischen Messgrößen von ein oder mehreren Fahrzeuginsassen erfolgen. Die somit erfolgende Bewertung verschiedener durchgeführter Fahrmanöver kann zur Erstellung ein oder mehrerer Benutzerprofile genutzt werden.

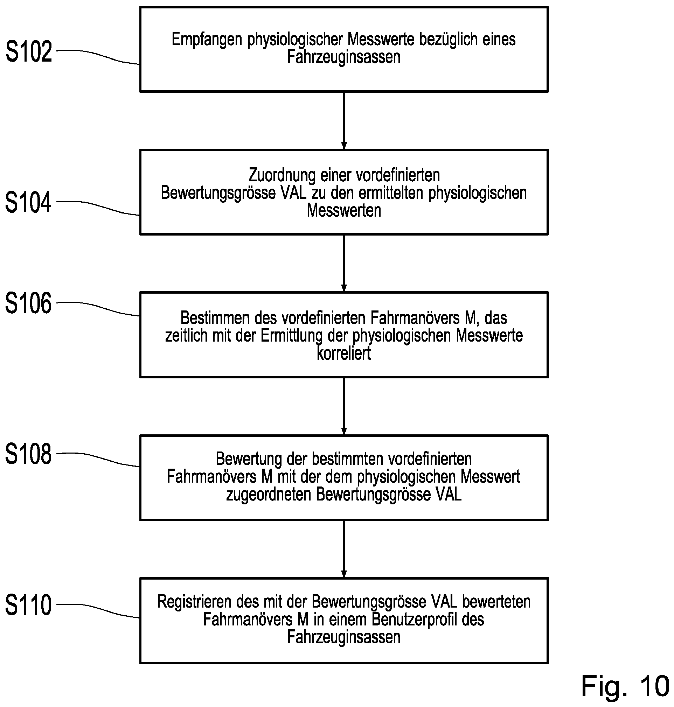

Fig. 10 zeigt ein Flussdiagramm, das die Erstellung eines Benutzerprofils durch die Steuereinheit für autonomes Fahren veranschaulicht. In einem Schritt S102 empfängt die Steuereinheit für autonomes Fahren physiologische Messwerte bezüglich eines Fahrzeuginsassen. In einem Schritt S104 ordnet die Steuereinheit für autonomes Fahren den erfassten physiologischen Messwerten jeweils eine vordefinierte Bewertungsgröße VAL zu. In einem Schritt S106 bestimmt die Steuereinheit für autonomes Fahren ein Fahrmanöver M, das zeitlich mit der Ermittlung der physiologischen Messwerte korreliert. In einem Schritt S108 bewertet die Steuereinheit für autonomes Fahren das erkannte Fahrmanöver M mit der dem physiologischen Messwert zugeordneten Bewertungsgröße VAL. In einem Schritt S110 registriert die Steuereinheit für autonomes Fahren das bewertete Fahrmanöver M in einem Benutzerprofil des Fahrzeuginsassen.

Im obigen Ausführungsbeispiel der Fig. 10 werden die physiologischen Messwerte absolut bewertet. In alternativen Ausführungsbeispielen können die physiologischen Messwerte aber auch relativ (auf Veränderungen basierend) bewertet werden. Nach einem abgeschlossenen Manöver können wie oben beschrieben die physiologischen Messwerte (Biodaten, Gestik und/oder Sprache von Fahrzeuginsassen) analysiert werden.

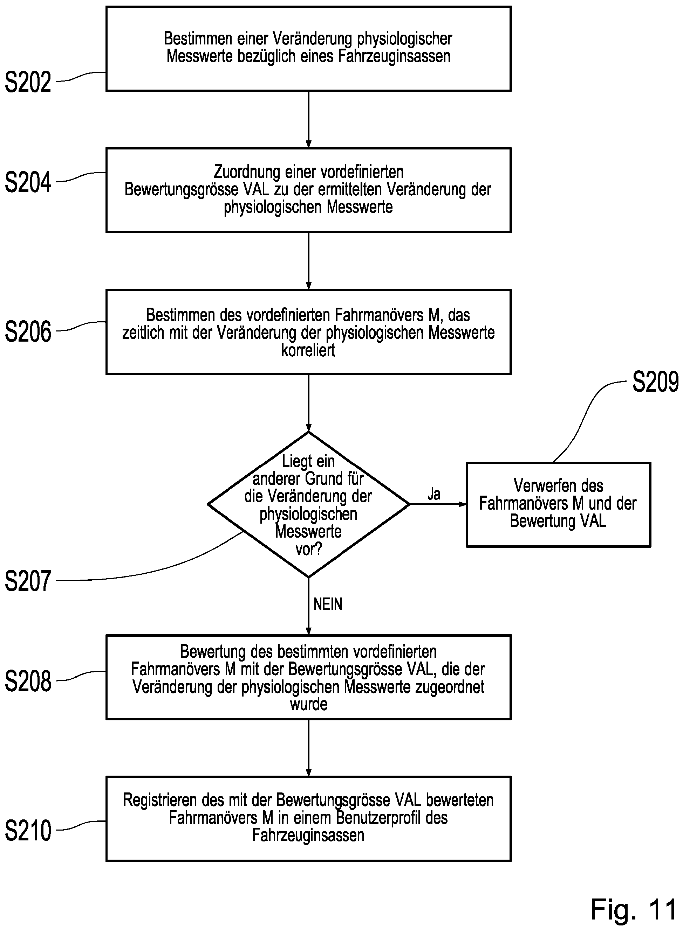

Fig. 11 zeigt ein Flussdiagramm, das ein Verfahren zur Erstellung eines Benutzerprofils durch die Steuereinheit für autonomes Fahren veranschaulicht, bei dem die physiologischen Messwerte relativ (auf Veränderungen basierend) bewertet werden. Nach einem Manöver kann z.B. die Herzfrequenz steigen wenn der Fahrzeuginsasse Angst bekommt, der Blutdruck kann sich verändern, die Körperhaltung passt sich dem Befinden an (Verkrampfen, Festhalten, entspannt "Sacken lassen", nervöses Verhalten, usw.), die Hautfarbe passt sich an und dergleichen. Die Steuereinheit für autonomes Fahren kann diese Information über Veränderungen der Befindlichkeit eines oder mehrerer Fahrzeuginsassen nutzen und die Fahrweise anpassen (z.B. mehr/weniger Abstand zum Vordermann, früher/später Kurven anbremsen, kleinere/größere Kurvengeschwindigkeiten, sportlichere Fahrweise).

In einem Schritt S202 bestimmt die Steuereinheit für autonomes Fahren eine Veränderung physiologischer Messwerte bezüglich eines Fahrzeuginsassen. In einem Schritt S204 ordnet die Steuereinheit für autonomes Fahren der ermittelten Veränderung der physiologischen Messwerte eine vordefinierte Bewertungsgröße VAL zu. Bei keiner bis geringer Veränderung der physiologischen Messwerte oder keinen auffälligen Gesten und Wörtern kann das Manöver entsprechend gut bewertet werden. Sollten sich die physiologischen Messwerte stärker verändert haben, wird analysiert, ob das Fahrmanöver der Grund für die Veränderung ist. In einem Schritt S206 bestimmt die Steuereinheit für autonomes Fahren ein Fahrmanöver M, das zeitlich mit der ermittelten Veränderung der physiologischen Messwerte korreliert. In einem Schritt S207 prüft die Steuereinheit für autonomes Fahren ob ein anderer Grund für die Veränderung der physiologischen Messwerte vorliegt. Wird in dem Schritt S207 festgestellt, dass kein anderer Grund vorliegt, so bewertet die Steuereinheit für autonomes Fahren in einem Schritt S208 das erkannte Fahrmanöver M mit der Bewertungsgröße VAL, die der ermittelten Veränderung der physiologischen Messwerte zugeordnet wurde. In einem Schritt S210 registriert die Steuereinheit für autonomes Fahren das bewertete Fahrmanöver M in einem Benutzerprofil des Fahrzeuginsassen. Wird im Gegenteil in dem Schritt S207 festgestellt, dass ein anderer Grund vorliegt, so werden in einem Schritt S209 das Fahrmanöver M und die Bewertung VAL verworfen.

Ebenso bei Gestik und Sprache kann der Ursprung geklärt werden. Sollte das Fahrmanöver der einzig mögliche Grund sein, kann anhand der Änderung bzw. Äußerung eine entsprechende Bewertung definiert werden. Wird dagegen mit Mitteln wie Bild- oder Sprachanalyse erkannt, dass die Insassen sich in einer emotional anstrengenden Konversation befinden, so kann die Steuereinheit für autonomes Fahren entscheiden, dass die zugehörigen physiologischen Messwerte nicht für die Bewertung von Fahrmanövern herangezogen werden.

Durch kontinuierliche Analyse aller Manöver für sämtliche Fahrzeuginsassen lassen sich Benutzerprofile mit Bewertungen aller Fahrmanöver erstellen. Dadurch wird es möglich, dass die Steuereinheit für autonomes Fahren vorhersieht, wie bestimmte Personengruppen bei bestimmten Manövern reagieren, d.h. wie die Manöver bei den Fahrzeuginsassen "ankommen". Insassen bewerten Fahrweisen unterschiedlich. Ziel kann es sein, die Fahrweise so anzupassen, dass unterschiedliche Personen sich immer sicher fühlen.

Auf Grundlage des auf diese Weise erstellten Benutzerprofils kann die Steuereinheit für autonomes Fahren nun ein anstehendes Fahrmanöver bzw. Fahrmanövervarianten bewerten. Die Wahl der passenden Fahrweise (Geschwindigkeiten, Querbeschleunigungen, Manöver: Ausweichen, Bremsen oder Kombination, Beschleunigungen und Verzögerungen, Position in der Fahrspur: links, rechts, mittig, ...) wird auf diese Weise vom autonomen Fahrzeug selbst durchgeführt.

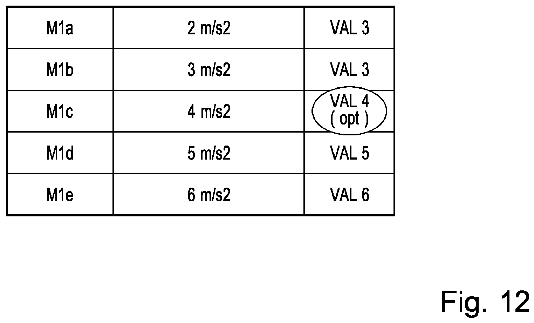

Fig. 12 zeigt eine Tabelle, die ein Benutzerprofil für die Fahrmanövervarianten M1a - M1e der Fahrmanöverkategorie M1 "Fahrt durch eine Kurve" gemäß dem vorliegenden Ausführungsbeispiel darstellt, das von der Steuereinheit 18 für autonomes Fahren erstellt wurde. Wie aus der Tabelle ersichtlich ist, wird einer Fahrmanövervariante M1a "Fahrt durch eine Kurve mit einer Querbeschleunigung von 2 m/s²" die vordefinierte Bewertungsgröße VAL3 als Bewertungsgröße zugeordnet; einer Fahrmanövervariante M1b "Fahrt durch eine Kurve mit einer Querbeschleunigung von 3 m/s²" wird die vordefinierte Bewertungsgröße VAL3 als Bewertungsgröße zugeordnet; einer Fahrmanövervariante M1c "Fahrt durch eine Kurve mit einer Querbeschleunigung von 4 m/s²" wird die vordefinierte optimale Bewertungsgröße VAL4 als Bewertungsgröße zugeordnet; einer Fahrmanövervariante M1d "Fahrt durch eine Kurve mit einer Querbeschleunigung von 5 m/s²" wird die vordefinierte Bewertungsgröße VAL5 als Bewertungsgröße zugeordnet (vgl. Fig. 8 und Fig. 9); und einer Fahrmanövervariante M1e "Fahrt durch eine Kurve mit einer Querbeschleunigung von 6 m/s²" wird die vordefinierte Bewertungsgröße VAL6 als Bewertungsgröße zugeordnet.

Das erstellte Benutzerprofil wird dann von der Steuereinheit für autonomes Fahren 18 benutzt, um anstehende Fahrmanöver so zu steuern, dass das Fahrverhalten des Fahrzeugs optimiert wird. Die Fahrweise wird so an die Personen im Fahrzeug angepasst. Demgemäß, wenn ein anstehendes Fahrmanöver der Fahrmanöverkategorie M1 "Fahrt durch eine Kurve" auszuführen ist, wählt die Steuereinheit für autonomes Fahren 18 die Fahrmanövervariante M1c "Fahrt durch eine Kurve mit einer Querbeschleunigung von 4 m/s²", welcher die vordefinierte optimale Bewertungsgröße VAL4 als Bewertungsgröße zugeordnet ist.

Die Steuereinheit 18 für autonomes Fahren gemäß der vorliegenden Erfindung kann das erstellte Benutzerprofil speichern und bei einer späteren Fahrt erneut nutzen, um das Fahrverhalten des Fahrzeugs zu optimieren.

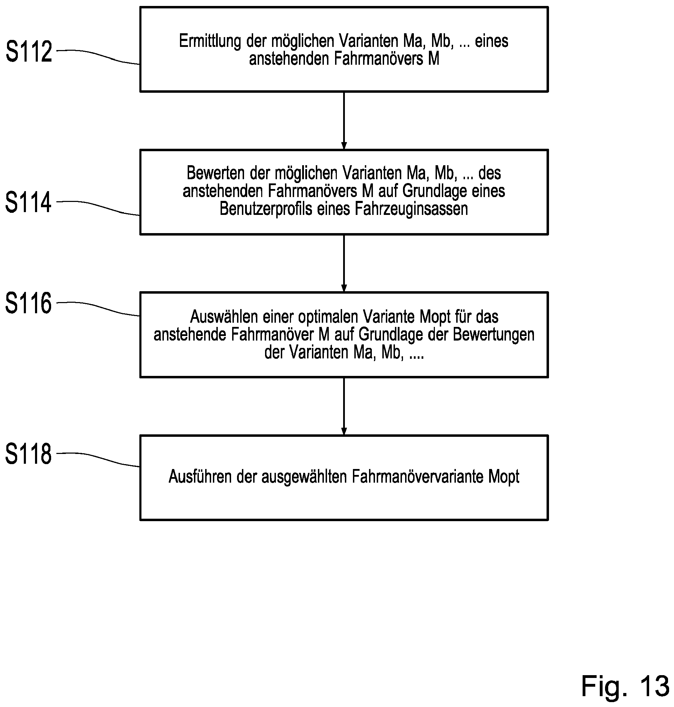

Fig. 13 zeigt ein Flussdiagramm, das die Bewertung durch die Steuereinheit für autonomes Fahren 18 von möglichen Fahrmanövervarianten eines anstehenden Fahrmanövers auf Grundlage eines Benutzerprofils eines Fahrzeuginsassen veranschaulicht. In einem Schritt 112 ermittelt die Steuereinheit für autonomes Fahren mögliche Varianten Ma, Mb, Mc usw. eines durchzuführenden Fahrmanövers M. In einem Schritt 114 werden diese Fahrmanövervarianten auf Grundlage eines zuvor erstellten Benutzerprofils eines Fahrzeuginsassen bewertet. In einem Schritt 116 wird dann auf Grundlage der Bewertungen der Fahrmanövervarianten eine optimalen Variante Mopt für das anstehende Fahrmanöver M ausgewählt. In einem Schritt 118 steuert die Steuereinheit für autonomes Fahren eine Steuereinheit für ein Lenksystem, eine Steuereinheit für ein Bremssystem und/oder eine Steuereinheit für einen Antriebstrang gemäß der ausgewählten optimalen Fahrmanövervariante so an, dass das ausgewählte Fahrmanöver ausgeführt wird.

Im obigen Ausführungsbeispiel wählt die Steuereinheit für autonomes Fahren jeweils die aus Sicht der Befindlichkeit des Insassen optimale Fahrmanövervariante aus. In alternativen Ausführungsbeispielen kann die Steuereinheit für autonomes Fahren abhängig von der Verkehrssituation auch eine Abwägung zwischen Befindlichkeit eines Fahrzeuginsassen und Anforderungen einer Verkehrssituation treffen. So kann die Steuereinheit für autonomes Fahren beispielsweise bei einem Überholvorgang entscheiden, dass es der Verkehrssituation angemessen ist, einen gewissen Zustand von Stress für den bzw. die Fahrzeuginsassen in Kauf zu nehmen, um den Überholvorgang zügig abzuschließen. So kann beispielsweise für jede Art von Fahrmanöver ein Parameter festgelegt werden, der bestimmt, welchen Grad an Einfluss die Insassenbefindlichkeit auf die Wahl der Fahrmanövervariante haben soll.

Aufgrund der Bewertung der Fahrmanöver bzw. aufgrund von Informationen zur Gesundheit eines Fahrzeuginsassen können bestimmte Manöver für bestimmte Insassen durch andere Manöver auch ersetzt werden. Beispielsweise kann die Steuereinheit für autonomes Fahren ein Hindernis auf der Straße erkennen und als anstehende Fahrmanövervarianten das "Vermeiden der Kollision mit dem Hindernis" erkennen. Für dieses Fahrmanöver kann die Steuereinheit für autonomes Fahren zwei mögliche Fahrmanövervarianten erkennen, nämlich als erste Fahrmanövervariante das "Umfahren des Hindernisses" und als zweite Fahrmanövervariante das "Abbremsen vor dem Hindernis". Je nach den Informationen im Benutzerprofil können dann bestimmte Manöver verhindert und durch andere mögliche Manöver ersetzt werden. Beispielsweise kann aus dem Benutzerprofil hervorgehen, dass ein Fahrzeuginsasse auf die Fahrmanövervariante "Umfahren des Hindernisses" in der Regel negativ reagiert, jedoch auf die Fahrmanövervariante "Abbremsen vor dem Hindernis" in der Regel positiv reagiert. Folglich kann die Steuereinheit für autonomes Fahren hier die bevorzugte Fahrmanövervariante "Abbremsen vor dem Hindernis" auswählen. Auf diese Weise ersetzt die Steuereinheit für autonomes Fahren beim betreffenden Fahrzeuginsassen die Fahrmanövervariante "Umfahren des Hindernisses" durch die Fahrmanövervariante "Abbremsen vor dem Hindernis".

In einem Benutzerprofil können auch Informationen zur Gesundheit eines Fahrzeuginsassen hinterlegt werden und bestimmte Fahrmanöver je nach Gesundheitszustand vermieden werden. Der Gesundheitszustand kann Einfluss auf die Bewertung eines Fahrmanövers nehmen. Die Fahrweise kann anhand der Gesundheitsdaten bewertet werden. Durch diese Daten kann die Fahrweise laufend optimiert werden. Wird beispielsweise von der Steuereinheit für autonomes Fahren erkannt, dass sich ein kranker Fahrzeuginsasse im Fahrzeug befindet, so werden von der Steuereinheit für autonomes Fahren systematisch solche Fahrmanöver schlechter bewertet, die den Fahrzeuginsassen beanspruchen, wie beispielsweise Kurvenfahrten mit hohen Querbeschleunigungen oder dergleichen. Auf diese Weise können Gesundheitsdaten Einfluss auf die Fahrweise des Fahrzeugs haben. Somit kann das System vorkonditioniert werden (Einstellen einer bestimmten Fahrweise bereits beim Einsteigen der Personen). Kranke, Alte, empfindliche Personen können besonders behutsam (wenig Quer- und Längsbeschleunigungen) ans Ziel gebracht werden. Fahrzeuginsassen, die im Stress sind, können durch eine bestimmte eingestellte und angepasste Fahrweise beruhigt werden. Musik und Beleuchtung im Fahrzeug können ebenfalls anhand der Gesundheitsdaten angepasst werden. Ziel ist es die Insassen zu beruhigen und sie sicher und zufrieden an ihr Ziel bringen.

Gemäß einem weiteren Aspekt der Erfindung können verletze Personen oder schwangere Frauen mit Wehen so schnell wie möglich auf kürzestem Weg ins Krankenhaus gebracht werden, soweit die Steuereinheit für autonomes Fahren durch Analyse der physiologischen Messwerte einen entsprechenden Gesundheitszustand erkennt.

Die durch die obigen Ausführungsbeispiele ermittelten Bewertungen von Fahrmanövern bzw. die auf dieser Grundlage erstellten Benutzerprofile können auch dazu dienen, die Fahrweise weiter zu optimieren, beispielsweise durch Verwendung von Deep Learning, Künstlicher Intelligenz (KI) oder Machine-Learning. Die Fahrweise kann beispielsweise schrittweise angepasst werden, z.B. in Stufen. Die Fahrweise kann so in Stufen unterteilt werden und schnell merklich angepasst werden. Die Fahrweise kann aber auch kontinuierlich angepasst werden, d.h. sie wird gemäß minimalen Schritten verändert. Auch kann die Fahrweise in Kombination schrittweise und kontinuierlich angepasst werden.

Die Steuereinheit für autonomes Fahren kann auch so ausgelegt sein, dass die laufende Optimierung der Fahrweise mittels einer Benutzerschnittstelle (HMI = Human-Machine-Interface) (Benutzerschnittstelle 26 in Fig. 2) ein oder ausgeschalten werden kann. Es kann beispielsweise von den Fahrzeuginsassen mittels Sprach-, Gestik-, Haptikeingabe oder dergleichen gewählt werden, ob schrittweise, kontinuierlich, beides oder gar nicht optimiert wird. Der Bereich der Optimierung kann von den Insassen beschränkt werden. Diese wählen z.B. über eine Benutzerschnittstelle eine "gemütliche/ökonomische Fahrweise". Dies kann beispielsweise bei der Zieleingabe für das autonome Fahrzeug mittels Sprach-, Gestik-, Haptikeingabe oder dergleichen erfolgen. So kann beispielsweise die Fahrweise beim Start gewählt werden. Die Steuereinheit für autonomes Fahren optimiert dann die Fahrweise entsprechend, indem sie "gemütlichere" bzw. "ökonomischere" Fahrmanöver durch bessere Bewertung priorisiert. Alternativ können die Fahrzeuginsassen eine Fahrweise "sportlich" wählen. Dann wird die Steuereinheit für autonomes Fahren sportlichere Fahrmanöver besser bewerten und dadurch priorisieren. Die Richtung der Optimierung wird von den Fahrzeuginsassen über die Benutzerschnittstelle vorgegeben. Mit diesen optionalen Einstellmöglichkeiten können ungewollte Optimierungen der Fahrweise durch die Steuereinheit für autonomes Fahren verhindert werden.

Die gewonnen Daten (Benutzerprofile) können auch aus der Steuereinheit für autonomes Fahren exportiert werden und weiter genutzt werden, um beispielsweise in anderen Fahrzeugen genutzt zu werden. So könnte das Benutzerprofil auch zentralisiert gespeichert werden, beispielsweise auf einem Server oder in einem Cloud-Dienst. Auch könnte ein Benutzerprofil der Steuereinheit für autonomes Fahren mit einem zentralen Benutzerprofil synchronisiert werden. Daten aus dem Benutzerprofil können beispielsweise für Langzeitstudien und weitere Analysen an den Hersteller übermittelt werden. Gesammelte Daten können als Trainingsdaten für KI Algorithmen verwendet werden. Das Fahrzeugsteuergerät oder - soweit Aspekte davon auf einen Server oder in die Cloud ausgelagert sind - das Benutzerendgerät können die Daten an eine zentrale Einheit (z.B. des Herstellers) übermitteln und diese kann somit Rückschlüsse über den Algorithmus ziehen, wie dieser bei einer großen Stichprobe ankommt und ob dieser bereits an den Grenzen seiner Leistungsfähigkeit angekommen ist (Fahrzeug soll sich anders verhalten hat aber keine Möglichkeit dieses anzupassen da es sich schon im Grenzbereich der Funktion befindet). Dies hat den Vorteil, dass der Hersteller dann den Algorithmus weiterentwickeln und ein Update für die Steuerlogik im Fahrzeug anbieten kann.

### Bezugszeichen

- 1: tragbares Benutzergerät
- 2: autonomes Fahrzeug
- 12: Steuereinheit für Lenksystem
- 14: Steuereinheit für Bremssystem
- 16: Steuereinheit für Antriebstrang
- 18: Steuereinheit für autonomes Fahren
- 19: Kommunikationsschnittstelle
- 20: Umfeldsensoren
- 21: Innenraumkamera
- 22: Kommunikationsschnittstelle
- 23: Mikrofon
- 24: Satellitennavigationseinheit
- 26: Benutzerschnittstelle
- 28: Fahrzeugkommunikationsnetzwerk
- 30: Prozessor tragbares Benutzergerät
- 31: Biosensor
- 32: RAM-Speicher
- 33: ROM-Speicher
- 34: Speicherlaufwerk
- 35: Benutzerschnittstelle
- 36: Kommunikationsschnittstelle
- 37: Energiequelle
- 40: Prozessor Steuereinheit für autonomes Fahren
- 42: RAM-Speicher
- 43: ROM-Speicher
- 44: Speicherlaufwerk
- 45: Benutzerschnittstelle

## Patentansprüche

1. Steuereinheit für autonomes Fahren (18), die einen Prozessor (40) umfasst, der dazu ausgelegt ist, ein durchgeführtes oder ein anstehendes Fahrmanöver (M1-M4) auf Grundlage von physiologischen Messwerten zu bewerten.

2. Steuereinheit für autonomes Fahren (18) nach Anspruch 1, wobei es sich bei den physiologischen Messwerten um eine Herzfrequenz, eine Hautleitfähigkeit, eine Sauerstoffsättigung im Blut, einen Blutdruck und/oder eine Adrenalinkonzentration eines Fahrzeuginsassen handelt.

3. Steuereinheit für autonomes Fahren (18) nach Anspruch 1, wobei es sich bei den physiologischen Messwerten um Informationen handelt, die durch Bildanalyse oder Sprachanalyse gewonnen werden.

4. Steuereinheit für autonomes Fahren (18) nach einem der vorstehenden Ansprüche, wobei der Prozessor (40) dazu ausgelegt ist, auf Grundlage von bewerteten Fahrmanövern ein Benutzerprofil zu erstellen.

5. Steuereinheit für autonomes Fahren (18) nach Anspruch 4, wobei der Prozessor (40) dazu ausgelegt ist, ein anstehendes Fahrmanöver auf Grundlage des erstellten Benutzerprofils zu bewerten.

6. Steuereinheit für autonomes Fahren (18) nach einem der vorstehenden Ansprüche, wobei der Prozessor (40) dazu ausgelegt ist, mehrere Varianten eines Fahrmanövers zu bewerten und eine optimale Variante auf Grundlage der Bewertung auszuwählen.

7. Steuereinheit für autonomes Fahren (18) nach einem der vorstehenden Ansprüche, wobei den physiologischen Messwerten unterschiedliche Gewichtungen beigemessen werden.

8. Steuereinheit für autonomes Fahren (18) nach einem der vorstehenden Ansprüche, wobei der Prozessor dazu ausgelegt ist, relative Veränderungen der physiologischen Messwerte zu erkennen und ein Fahrmanöver auf Grundlage der relativen Veränderungen der physiologischen Messwerte zu bewerten.

9. Steuereinheit für autonomes Fahren (18) nach einem der vorstehenden Ansprüche, wobei der Prozessor dazu ausgelegt ist, zu prüfen, ob eine Veränderungen der physiologischen Messwerte auf ein Fahrmanöver oder einen anderen Grund zurückzuführen ist und eine Bewertung des Fahrmanövers nur dann vorzunehmen, wenn das Fahrmanöver als Grund anzunehmen ist.

10. Verfahren für autonomes Fahren, bei dem ein durchgeführtes oder ein anstehendes Fahrmanöver auf Grundlage von physiologischen Messwerten bewertet wird.
